# EUROPEAN PATENT APPLICATION

(11) **EP 4 023 328 A1**
(43) Date of publication of application: **06.07.2022**
(21) Application number: 20870997.2
(22) Date of filing: 30.09.2020
(51) Int. Cl.: B01J 19/12, A23L 3/34, A61K 9/06, A61K 9/08, A61K 33/00, A61K 33/40, A61K 41/00, A61K 41/10, A61L 2/14, A61L 2/16, A61L 2/18, A61P 35/00, C01B 11/02, A61L 101/06

(54) **RADICAL MANUFACTURING METHOD, SPORE STERILIZATION METHOD, AND CANCER TREATMENT DRUG**

(30) Priority: 01.10.2019 JP 2019181758; 21.05.2020 JP 2020089254
(71) Applicant: Acenet Inc., Tokyo 105-0021 (JP)
(72) Inventor: KONISHI Kiyoshi, Tokyo 105-0021 (JP); TAKAMORI Kiyoto, Tokyo 105-0021 (JP); SHIBATA Takekatsu, Tokyo 105-0021 (JP)
(74) Representative: J A Kemp LLP
(86) International application number: PCT/JP2020/037371
(87) International publication number: WO 2021/066081

(57) **Abstract**

Provided is a novel method that makes it possible to easily sterilize spores and the like in a highly safe manner. A method for producing radicals according to the present invention includes a treatment step of generating radicals through photoirradiation of a radical generation source and treating spores with the radicals, and the peak wavelength of light used in the photoirradiation is greater than UV wavelengths and 600 nm or less. Also, a method for sterilizing spores according to the present invention includes a treatment step of generating radicals through photoirradiation of a radical generation source and treating spores with the radicals, and the peak wavelength of light used in the photoirradiation is greater than UV wavelengths and 600 nm or less. The peak wavelength is, for example, 405 to 470 nm.

## Description

### TECHNICAL FIELD

The present invention relates to a method for producing radicals, a method for sterilizing spores, and a cancer treatment drug.

### BACKGROUND ART

Various disinfectants and bactericides are used to prevent infection and the like. However, some bacteria (called spore-forming bacteria or spore-bearing bacteria) form spores, whose cell structures are extremely durable. For example, Bacillus anthracis, Clostridium tetani, and Clostridium boturinum are well known spore-forming bacteria. These spore-forming bacteria exist in a vegetative state when growing, but in a bad environment such as a dry environment, a high-temperature environment, or in the presence of a growth inhibiting substance or radioactive rays, the spore-forming bacteria stop growing and survive by changing into spores that exchange hardly any nutritive substances or the like with the external environment. When a normal environment is restored, the spores change to the vegetative state and start to grow again. Therefore, it is difficult to sterilize the spore-forming bacteria in the true sense of the word, namely, to inhibit the initiation of growth of the spore-forming bacteria in a normal environment, by using a common alcohol-based disinfectant or through heating, for example (Non Patent Literature 1).

At present, the use of glutaraldehyde, peracetic acid, or the like is known as a method for sterilizing spore-forming bacteria. However, these substances are highly toxic to cells and tissues, for example, and cannot be used on a living body, for example, and also cannot be used for medical instruments or the like made of metal because these substances cause metal corrosion.

### Citation List

### Non Patent Literature

[Non Patent Literature 1] M. J. Leggett, G. McDonnell, S. P. Denyer, P. Setlow, and J.-Y. Maillard, Bacterial spore structures and their protective role in biocide resistance. Journal Applied Microbiology, 113: 485-498 (2012)

### SUMMARY OF INVENTION

### Technical Problem

Therefore, an object of the present invention is to provide a novel method that makes it possible to easily sterilize spores and the like in a highly safe manner, for example.

### Solution to Problem

In order to achieve the above object, a production method for producing radicals according to the present invention includes:
a treatment step of generating radicals through photoirradiation of a radical generation source and treating spores with the radicals,
wherein a peak wavelength of light used in the photoirradiation is greater than UV wavelengths and 600 nm or less.

A sterilization method for sterilizing spores according to the present invention includes:
a treatment step of generating radicals through photoirradiation of a radical generation source and treating spores with the radicals,
wherein a peak wavelength of light used in the photoirradiation is greater than UV wavelengths and 600 nm or less.

A sterilization apparatus for sterilizing an instrument according to the present invention includes:
a reagent housing portion;
an instrument housing portion;
a connector; and
a photoirradiation portion,
wherein the instrument housing portion and the reagent housing portion are connected to the connector such that a reagent housed in the reagent housing portion is introduced into the instrument housing portion,
the reagent housing portion is configured to house a reagent that contains a radical generation source,
the instrument housing portion is configured to house an instrument to be sterilized,
the photoirradiation portion includes a light source configured to emit light toward at least one of the reagent housing portion and the instrument housing portion, and
a peak wavelength of light emitted from the light source is greater than UV wavelengths and 600 nm or less.

A cancer treatment drug according to the present invention includes a radical generation source,
wherein cancer cells are treated with radicals generated from the radical generation source.

### Advantageous Effects of Invention

As a result of intensive studies, the inventors of the present invention found that it is possible to generate radicals from a radical generation source by merely irradiating the radical generation source with light whose peak wavelength is greater than UV wavelengths and 600 nm or less, rather than UV rays that affect human bodies, nature, and the like, for example. According to the present invention, it is possible to generate radicals from a radical generation source by merely performing photoirradiation using light whose peak wavelength is greater than UV wavelengths and 600 nm or less, and spores can be sterilized with the generated radicals. Conditions of the photoirradiation are not conditions that are harsh on the environment, living bodies, and the like, and therefore, spores can be sterilized in a highly safe manner for the environment or an instrument to be treated, an operator, or the like.

### BRIEF DESCRIPTION OF DRAWINGS

[FIG. 1] FIG. 1 is a graph obtained when the number of colonies of Bacillus subtilis spores was checked in Example 1.
[FIG. 2] FIG. 2 includes a graph showing the molar concentration of ClO₂⁻ in Example 2.
[FIG. 3] FIG. 3 is a graph showing changes in the chlorine dioxide radical concentration and the oxygen concentration over time in Example 3.
[FIG. 4] FIG. 4 is a graph showing a relationship between the maximum value of the molar concentration of chlorine dioxide radicals and the maximum value of the molar concentration of oxygen in Example 3.
[FIG. 5] FIG. 5 is a schematic diagram showing a sterilization apparatus according to the present invention.

### DESCRIPTION OF EMBODIMENTS

The following describes the present invention more specifically using examples. However, the following description does not limit the present invention.

### Method for producing radicals

As described above, the method for producing radicals according to the present invention includes a generation step of generating radicals through photoirradiation of a radical generation source, and a peak wavelength of light used in the photoirradiation is greater than UV wavelengths and 600 nm or less.

### (1) Radical generation source

The radical generation source is not particularly limited and is only required to generate radicals under non-solid conditions, for example. Specifically, the radical generation source is a generation source that generates radicals when irradiated with light, for example. For example, WO2017/104797 is applicable in terms of the radical generation source and a radical generation catalyst, which will be described later.

The radical generation source may include, for example, at least one selected from the group consisting of halogen ions, hypohalous acid ions, halous acid ions, halogen acid ions, and perhalogen acid ions, and preferably includes halous acid ions. As the halous acid ions, for example, chlorite ions are preferable. The radical generation source may be any of the abovelisted ions or a salt of the ions, for example. Examples of the salt of the ions include a sodium salt and a potassium salt.

The radical generation source may include, for example, an oxoacid or a salt thereof, and specific examples thereof include halogen oxoacids and salts thereof. Examples of the oxoacid include boric acid, carbonic acid, orthocarbonic acid, carboxylic acid, silicic acid, nitrous acid, nitric acid, phosphorous acid, phosphoric acid, arsenic acid, sulfurous acid, sulfuric acid, sulfonic acid, sulfinic acid, chromic acid, dichromic acid, and permanganic acid. Examples of the halogen oxoacids include: chlorine oxoacids such as hypochlorous acid, chlorous acid, chloric acid, and perchloric acid; bromine oxoacids such as hypobromous acid, bromous acid, bromic acid, and perbromic acid; and iodine oxoacids such as hypoiodous acid, iodous acid, iodic acid, and periodic acid.

The radical generation source may include, for example, electron donor-acceptor linked molecules. There is no particular limitation on the electron donor-acceptor linked molecules, and the electron donor moiety may be one or more electron donating substituents and the electron acceptor moiety may be one or more aromatic cations. The aromatic cation may be a monocyclic cation or a fused ring cation, and the aromatic ring may optionally include a heteroatom, and may optionally include a substituent other than the electron donating substituent. There is no particular limitation on the number of atoms constituting the aromatic ring that forms the aromatic cation, and the aromatic ring is, for example, a 5 to 26-membered ring.

Examples of the aromatic ring forming the aromatic cation include a pyrrolinium ring, a pyridinium ring, a quinolinium ring, an isoquinolinium ring, an acridinium ring, a 3,4-benzoquinolinium ring, a 5,6-benzoquinolinium ring, a 6,7-benzoquinolinium ring, a 7,8-benzoquinolinium ring, a 3,4-benzoisoquinolinium ring, a 5,6-benzoisoquinolinium ring, a 6,7-benzoisoquinolinium ring, a 7,8-benzoisoquinolinium ring, and a ring in which at least one of the carbon atoms constituting any of these rings is substituted with a heteroatom.

### (2) Radical generation catalyst

Photoirradiation performed in the generation step of the present invention may be performed in a state where a radical generation catalyst is present together with the radical generation source.

There is no particular limitation on the radical generation catalyst, and examples thereof include a radical generation catalyst that contains at least one of ammonium and a salt thereof and a radical generation catalyst that contains an organic compound that includes at least one of a Lewis acid and a Brønsted acid.

Examples of the radical generation catalyst include cationic surfactants such as quaternary ammonium-type cationic surfactants. Examples of the quaternary ammonium-type cationic surfactants include benzalkonium chloride, benzethonium chloride, cetylpyridinium chloride, hexadecyltrimethylammonium bromide, dequalinium chloride, edrophonium, didecyldimethylammonium chloride, tetramethylammonium chloride, tetrabutylammonium chloride, benzyltriethylammonium chloride, oxitropium, carbachol, glycopyrronium, safranin, sinapine, tetraethylammonium bromide, hexadecyltrimethylammonium bromide, suxamethonium, sphingomyelin, denatonium, trigonelline, neostigmine, paraquat, pyridostigmine, Phellodendrine, pralidoxime iodide, betaine, betanin, bethanechol, betalain, lecithin, and cholines (choline chlorides such as benzoylcholine chloride and lauroylcholine chloride hydrate, phosphocholine, acetylcholine, choline, dipalmitoylphosphatidylcholine, choline bitartrate, etc.).

### (3) Photoirradiation

In the production method of the present invention, the generation step is a step of generating radicals through photoirradiation of the radical generation source, and the peak wavelength of light used in the photoirradiation is greater than UV wavelengths and 600 nm or less as described above. The photoirradiation of the radical generation source may be performed in a state where the radical generation catalyst is present together with the radical generation source, for example.

According to the present invention, the peak wavelength is within the above range, and therefore, it is possible to generate a product such as radicals from the radical generation source through the photoirradiation while sufficiently suppressing an influence on the environment and the human body, for example. The generated radicals can be used for various applications in which radicals are used, for example. The description of a method for sterilizing spores, which will be described later, is applicable to conditions and the like of the photoirradiation.

Examples of the applications include sterilization of spores, which will be described later. Application of the present invention is not limited to this example, and the present invention is also applicable to sterilization of fungi, bacteria, viruses, and the like. There is no particular limitation on the fields to which the present invention is applied, and the present invention can be used in fields in which sterilization treatment is performed, for example. Examples of such fields include various fields such as medical fields, food fields, agricultural fields, dairy fields, and processing fields.

Examples of fungi include Basidiomycota (mushrooms), Ascomycota (yeast and mold), Blastocladiomycota, and Chytridiomycota. When the present invention is applied to agricultural fields, examples of sterilization targets that belong to the Basidiomycota include Rigidoporus microporus that causes root rot of gum trees, cacao, cassava, and the like, and smut fungus that causes smut in wheat and barley, and examples of sterilization targets that belong to the Ascomycota include Mycosphaerella fijiensis that causes sigatoka disease of bananas and the like, Fusarium that causes wilt disease of bananas, tomatoes, sweet potatoes, beans, and the like (e.g., F. oxysporum that causes Panama disease in bananas), Fusarium that causes rot (e.g., F. oxysporum that causes fusarium basal rot of onions, F. solani that causes potato dry rot, and F. Fujikuroi that causes bakanae disease in rice), and ascomycete (e.g., Erysiphaceae) that causes mildew and the like. When the present invention is applied to the agricultural fields, the method of the present invention may be performed on plants themselves, or on soil, for example.

### Sterilization method

As described above, the method for sterilizing spores according to the present invention includes a treatment step of generating radicals through photoirradiation of the radical generation source and treating spores with the radicals, and the peak wavelength of light used in the photoirradiation is greater than UV wavelengths and 600 nm or less.

In the present invention "sterilizing spores" means reducing the number of spores that have the ability to grow.

### (1) Spores

There is no particular limitation on spores to which the present invention is applicable, and examples thereof include spores of spore-bearing bacteria. Examples of the spore-bearing bacteria include gram-positive bacilli such as the genus Bacillus and the genus Clostridium. Examples of the genus Bacillus include Bacillus anthracis, Bacillus cereus, and Bacillus subtilis, and examples of the genus Clostridium include Clostridium tetani, Clostridium boturinum, Clostridium perfringens, and Clostridium difficile.

### (2) Treatment step

In the sterilization method of the present invention, the treatment step is a step of generating radicals through photoirradiation of the radical generation source and treating spores with the radicals. The photoirradiation of the radical generation source may be performed in a state where the radical generation catalyst is present together with the radical generation source, for example.

As described above, the peak wavelength of light used in the photoirradiation is greater than UV wavelengths and 600 nm or less. In the present invention, UV (ultraviolet rays) refers to light that has wavelengths of 100 nm to 380 nm. According to the present invention, the peak wavelength is within the above range, and therefore, it is possible to sterilize highly durable spores by generating a product such as radicals from the radical generation source through the photoirradiation while sufficiently suppressing the impact on the environment and the human body, for example. Irradiation with ultraviolet rays whose peak wavelength is a UV wavelength is commonly performed as a sterilization treatment in laboratories, for example, and a clean bench or the like is used and a shield or the like is provided when performing the photoirradiation to shield the external environment from the ultraviolet rays in view of their influence on living bodies and the environment. However, according to the present invention, the peak wavelength is within the above range, and accordingly, there is no need to provide a shield, for example. Moreover, spores cannot be sufficiently killed through ultraviolet irradiation, but according to the present invention, spores can be killed as shown in the Examples, which will be described later, without the risks involved in the ultraviolet irradiation.

The lower limit of the peak wavelength of the light used in the photoirradiation is greater than the UV wavelengths described above, specifically, more than 380 nm, for example, preferably 400 nm or more, or more than 400 nm, and more preferably 405 nm or more, 415 nm or more, 440 nm or more, or 455 nm or more in terms of being able to sufficiently suppress influence on the environment and living bodies, for example. The upper limit of the peak wavelength of the light used in the photoirradiation is 600 nm or less, for example, 520 nm or less, 500 nm or less, or 480 nm or less, and preferably 470 nm or less, 465 nm or less, or 460 nm or less in terms of being able to sufficiently generate from the radical generation source a product, such as radicals, that is effective for sterilizing spores, for example. The lower limit of the range of wavelengths included in the light used in the photoirradiation is more than 380 nm, for example, 400 nm or more, or more than 400 nm, and more preferably 405 nm or more, 415 nm or more, or 440 nm or more for the same reason as that described for the peak wavelength. The upper limit of the range of wavelengths included in the light used in the photoirradiation is 600 nm or less, 520 nm or less, 500 nm or less, or 480 nm or less, for example, and for the same reason as that described for the peak wavelength, the upper limit is 470 nm or less, 465 nm or less, or 460 nm or less, for example. A spectral line half width (full width at half maximum) of the light used in the photoirradiation is, for example, 2 to 100 nm, 5 to 50 nm, or 12 to 35 nm.

There is no particular limitation on the type of light source of the light used in the photoirradiation, and an LED can be used, for example. When the LED is used, the peak wavelength and the spectral line half width of the LED are the same as those described above, for example.

There is no particular limitation on the irradiance of the light used in the photoirradiation, and the irradiance is, for example, 1 to 500 mW/cm², 2 to 300 mW/cm², or 100 to 200 mW/cm². There is no particular limitation on the radiant energy of the light used in the photoirradiation, and the radiant energy is, for example, 0.05 to 4000 J/cm², 1 to 2000 J/cm², or 100 to 400 J/cm².

The treatment step is performed using a first method or a second method, for example. First, the first method will be described. In the first method, the treatment step includes a step of generating radicals by irradiating the radical generation source with light in the absence of spores and a step of treating spores with the radicals. That is, the first method is a method of generating radicals in advance and then treating spores with the radicals.

There is no particular limitation on conditions such as the temperature and the period of the photoirradiation performed in the absence of spores, and the conditions can be set as appropriate according to the type of spores to be treated later, the type of a treatment target in which spores may exist, the type of the radical generation source, and the like, for example. There is no particular limitation on the temperature, and the temperature is, for example, 0°C to 100°C or 4°C to 50°C. So-called room temperature may be adopted. There is no particular limitation on the period, and the period is, for example, 1 minute to 48 hours, or 1 minute to 24 hours.

There is no particular limitation on the form of a reagent of the radical generation source, and examples thereof include fluid forms. In the case where the reagent is a fluid reagent that contains the radical generation source, the reagent may be a liquid or a gel, and is preferably a liquid from the standpoint of ease of use. The liquid may be used as a mist by being sprayed, for example, or may be formed into nano particles and used in a dispersed state. The fluid reagent can be prepared by mixing the radical generation source and a solvent (fluid solvent), for example, and examples of the solvent include water (e.g., purified water, ion exchange water, pure water, or sterilized water), a buffer solution, alcohol such as ethanol, and an acetic acid solvent. There is no particular limitation on the buffer solution, and examples thereof include a potassium phosphate buffer solution. The above solvents may be used alone or in combinations of two or more of them, for example. There is no particular limitation on the pH of the fluid reagent, and the pH is, for example, 5 to 10, 6 to 9, or 7 to 8. The acetic acid solvent also functions as a Lewis acid or a Brønsted acid of the second radical generation catalyst, as well as the solvent, for example.

In the reagent, the radical generation source may be optionally dissolved in the solvent, or may be dispersed, suspended, sedimented, or diffused in the solvent, for example. In the case where the reagent is a gel, the radical generation source may be in the colloidal state, for example.

There is no particular limitation on the concentration of the radical generation source in the reagent, and the concentration is, for example, 0.5 to 80 mmol/L, 1 to 40 mmol/L, or 4 to 15 mmol/L.

When the radical generation catalyst is used together with the radical generation source, the reagent containing the radical generation source may further contain the radical generation catalyst, or the reagent containing the radical generation source may be used together with a reagent that contains the radical generation catalyst. When the radical generation source and the radical generation catalyst are used together, the molar ratio between the radical generation source and the radical generation catalyst is, for example, 1:0.01 to 100, 1:0.1 to 50, or 1: 1 to 5.

The reagent may further contain other substances, for example, and examples of the other substances include a pH adjusting agent and a buffer agent.

There is no particular limitation on the amount of the reagent added to the target in the first method, and the amount can be set as appropriate according to the type of target, the type of spores to be sterilized, and the like. The addition amount of the reagent can be expressed indirectly using the amount of the radical generation source contained in the reagent before the photoirradiation, and is, for example, 0.1 to 100 µmol, 1 to 20 µmol, or 5 to 10 µmol per 1 cm² of the target. In a case where the target is a liquid, the addition amount of the reagent can be expressed indirectly using the amount of the radical generation source contained in the reagent before the photoirradiation, and is, for example, 0.5 to 500 µmol, 5 to 100 µmol, or 25 to 50 µmol per 1 cm³ of the target.

In the treatment step of the first method, first, the reagent that does not contain spores is irradiated with light to generate radicals in the reagent, for example. Then, the radical-containing reagent in which radicals have been generated is added to a target in which spores exist (including a target in which spores may exist) to treat spores in the target with the radicals. Even when it is difficult to irradiate the target with light, for example, this method makes it possible to treat spores in the target with radicals by adding, to the target, the radical-containing reagent in which the radicals have been generated in advance. Specifically, this method is useful when a light source to be used for the photoirradiation is not available in the place where the target is present, for example.

There is no particular limitation on the method for adding the radical-containing reagent to the target, and the reagent may be dripped into the target, applied to the target, sprayed or scattered over the target, the target may be immersed in the reagent, or a space in which the target is placed may be filled with the reagent, for example. Besides the above, it is also possible to use a method of impregnating a carrier such as gauze or absorbent cotton with the reagent and bringing the carrier into contact with the target, for example. There is no limitation on the target in which spores exist, and examples thereof include solids, liquids, and semisolids. The present invention is applicable to various targets and specific examples thereof include objects and places that may be touched by people (e.g., a door handle, a table, goods used around a bed, clothes, a toilet, a car, a bath, etc.), tools that are used by people (e.g., a chopping board, a kitchen knife, etc.), machines (e.g., agricultural equipment), a humidifier, food and drink, soil, agricultural land, water, wastewater, and a swimming pool.

Next, the second method will be described. In the second method, the treatment step includes a step of generating radicals by irradiating the radical generation source with light in the presence of spores and treating the spores with the radicals. That is, the second method is a method of generating radicals while treating spores with the radicals at the same time.

There is no particular limitation on conditions such as the temperature and the period of the photoirradiation performed in the presence of spores, and the conditions can be set as appropriate according to the type of target spores, the type of a treatment target in which spores may exist, the type of the radical generation source, and the like, for example. The temperature is, for example, 0°C to 100°C or 4°C to 50°C, and so-called room temperature may be adopted. The period is, for example, 1 minute to 24 hours.

There is no particular limitation on the form of a reagent of the radical generation source, and examples thereof include fluid forms. The examples described for the first method are applicable to the second method.

There is no particular limitation on the concentration of the radical generation source in the reagent, and the concentration is, for example, 0.5 to 80 mmol/L, 1 to 20 mmol/L, or 2 to 7 mmol/L.

In the second method, first, the reagent is added to a target in which spores exist (including a target in which spores may exist), and the above-described photoirradiation is performed on the reagent, for example.

There is no particular limitation on the method for adding the reagent to the target, and the examples described for the first method are applicable to the second method.

With the second method, it is possible to generate radicals and treat spores in the target with the generated radicals by dripping the reagent onto the target, for example, and irradiating the reagent with light. Therefore, the treatment can be performed more easily, for example. Also, in the second method, an effective product generated through the photoirradiation of the reagent immediately acts on the spores, for example, and accordingly, the amount of the product (e.g., radicals) that disappears before acting on the spores can be reduced when compared with the first method, for example, and therefore, it is possible to reduce the amount of the radical generation source contained in the reagent. Examples of effective components generated through the photoirradiation of the reagent other than the radicals include singlet oxygen. In the second method, in addition to the radicals, singlet oxygen that has a short half-life immediately acts on the spores, and therefore, even when the concentration of the radical generation source is set lower than that in the second method, the spores can be efficiently sterilized in a similar manner. Therefore, a cost can be reduced, for example. There is no limitation on the method for measuring singlet oxygen, and the singlet oxygen can be measured by, for example, placing, into a vial, a sample that has a total volume of 650 µL and is prepared by adding NaClO₂ powder such that its final concentration is 14.5 mmol/L, 13 µL of a 1 mol/L potassium phosphate buffer solution, and 637 µL of D₂O, irradiating the sample with light having a wavelength of 405 nm using a measurement device (QE-5000 manufactured by Otsuka Electronics Co., Ltd.), and confirming emission of light having a wavelength of 1270 nm.

There is no particular limitation on the amount of the radical generation source added to the target in the second method, and the amount can be set as appropriate according to the type of target, the type of spores to be sterilized, and the like. The addition amount of the radical generation source is, for example, 0.1 to 100 µmol, 1 to 10 µmol, or 3 to 5 µmol per 1 cm² of the target. In a case where the target is a liquid, the addition amount of the radical generation source is, for example, 0.5 to 500 µmol, 5 to 50 µmol, or 15 to 30 µmol per 1 cm³ of the target.

There is no particular limitation on fields to which the present invention can be applied, and the present invention can be used in fields in which it is necessary or advantageous to sterilize spores. Examples of such fields include various fields such as medical fields, food fields, agricultural fields, dairy fields, and processing fields. In the medical fields, it is possible to sterilize the site of operation, surgical instruments such as a photogastroscope, a catheter, a nebulizer, a scalpel, and a laparoscope, a dialyzer, collected blood, and body wastes such as urine, for example. The sterilization method of the present invention is also applicable to, for example, living bodies, and examples thereof include humans and non-human animals excluding humans. There is no particular limitation on the site of the living body to which the reagent is added. For example, the reagent is added to a site in which spores may exist, and specific examples thereof include skin, cells, tissues, and organs. The sterilization method of the present invention may be applied in vivo or in vitro, for example.

When the radical generation source is irradiated with light, radicals, singlet oxygen, superoxide ions, hydrogen peroxide, and the like are generated.

### Method for treating spore infection

An infection treatment method for treating spore infection according to the present invention includes a treatment step of generating radicals through photoirradiation of the radical generation source and treating an affected part with the radicals, and the peak wavelength of light used in the photoirradiation is greater than UV wavelengths and 600 nm or less. The above description of the sterilization method of the present invention is applicable to the infection treatment method of the present invention.

Similarly to the sterilization method of the present invention, in the infection treatment method of the present invention, the treatment step may be performed using either: a first method in which the treatment step includes a step of generating radicals by irradiating the radical generation source with light in the absence of the affected part and a step of treating the affected part by administering the radicals to the affected part; or a second method in which radicals are generated and at the same time the affected part is treated with the radicals in the treatment step. That is, it can be said that the first method is a method of generating radicals in advance in a reagent that contains the radical generation source, and administering the reagent containing the radicals to the affected part to sterilize spores in the affected part with the radicals, and the second method is a method of administering a reagent that contains the radical generation source to the affected part and generating radicals through photoirradiation while at the same time sterilizing spores in in the affected part with the generated radicals.

In the present invention, "treatment" encompasses preventing infection of an affected part by sterilizing spores, suppressing the spread of infection by sterilizing spores in an affected part that is infected, and curing infection, and may refer to any of these according to the purpose. In the present invention, an "affected part" encompasses not only a part that has already been infected, but also a part that has the potential to be infected, and may refer to either of these according to the purpose.

In the present invention, an administration target is a human or a non-human animal excluding humans, for example. Examples of the non-human animal include a mouse, a rat, a rabbit, a monkey, a dog, and cattle. There is no particular limitation on the affected part, and examples thereof include skin, cells, tissues, and organs.

### Sterilization apparatus

A sterilization apparatus for sterilizing an instrument according to the present invention includes a reagent housing portion, an instrument housing portion, a connector, and a photoirradiation portion. The instrument housing portion and the reagent housing portion are connected to the connector such that a reagent housed in the reagent housing portion is introduced into the instrument housing portion. The reagent housing portion can house a reagent that contains a radical generation source, and the instrument housing portion can house an instrument that is to be sterilized. The photoirradiation portion includes a light source that emits light toward at least one of the reagent housing portion and the instrument housing portion. The peak wavelength of light emitted from the light source is greater than UV wavelengths and 600 nm or less.

The sterilization apparatus of the present invention is an apparatus for performing the predetermined photoirradiation described above, and accordingly, can be used for spore sterilization treatment of instruments as described above, for example. The sterilization target is not limited to spores, and the apparatus can also be used to sterilize various bacteria, fungi, viruses, and the like, as well as spores.

The reagent housed in the reagent housing portion is only required to contain the radical generation source. There is no limitation on the form of the reagent, and the above description of the spore sterilization method of the present invention is applicable. The reagent is preferably a fluid reagent in which the radical generation source is contained in the fluid solvent. In the case where the reagent is a fluid reagent, the fluid reagent that already contains the radical generation source may be housed in the reagent housing portion, or the fluid reagent may be prepared in the reagent housing portion, for example. In the latter case, for example, the reagent housing portion houses the fluid solvent, and the fluid reagent containing the radical generation source can be obtained by adding the radical generation source to the fluid solvent housed in the housing portion. Specifically, it is possible to prepare the fluid reagent containing the radical generation source in the reagent housing portion by housing the fluid solvent in the reagent housing portion and adding the radical generation source into the reagent housing portion. With this method, the fluid reagent can be prepared easily when the radical generation source is a solid, for example. The reagent housing portion may further include a stirring portion, for example, and by operating the stirring portion in a state where the fluid solvent and the radical generation source are housed, it is possible to effectively prepare the fluid reagent containing the radical generation source.

There is no particular limitation on the type of instruments that can be sterilized using the sterilization apparatus of the present invention, and the instrument housing portion is only required to have a size and a shape that allow instruments that are potential sterilization targets to be housed in the instrument housing portion, for example. Examples of such instruments include endoscopes such as a laparoscope and a thoracoscope, a photogastroscope, and dialysis instruments (a blood purifier, a tube, etc.). The sterilization apparatus of the present invention may be installed on an instrument that is used in surgery or the like, for example.

There is no particular limitation on the position at which the light source is arranged in the sterilization apparatus of the present invention, and the light source is only required to be arranged at a position from which light can be emitted toward the inside of at least one of the reagent housing portion and the instrument housing portion. In a case where the light source is arranged at a position from which light can be emitted toward the reagent housing portion, for example, first, a fluid reagent containing the radical generation source is housed in the reagent housing portion, the fluid reagent housed in the reagent housing portion is irradiated with light emitted from the light source, and the fluid reagent containing radicals generated through the photoirradiation is introduced via the connector into the instrument housing portion in which the instrument is housed. In a case where the light source is arranged at a position from which light can be emitted toward the instrument housing portion, for example, first, a fluid reagent containing the radical generation source is housed in the reagent housing portion, and then introduced via the connector into the instrument housing portion in which the instrument is housed, and the fluid reagent introduced into the instrument housing portion is irradiated with light emitted from the light source. Thus, sterilization treatment of the instrument housed in the instrument housing portion can be performed with the radicals.

In the sterilization apparatus of the present invention, the reagent housing portion or the instrument housing portion, which is irradiated with light to generate radicals, may be optionally provided with a light shield, for example. In the case of UV irradiation, a shield is usually arranged such that UV rays do not reach an operator, because UV rays are known to affect human bodies and the environment, for example. On the other hand, the light shield does not necessarily have to be provided in the present invention because the predetermined light that is highly safety, is used. Accordingly, for example, a production cost can be suppressed with the use of the sterilization apparatus of the present invention.

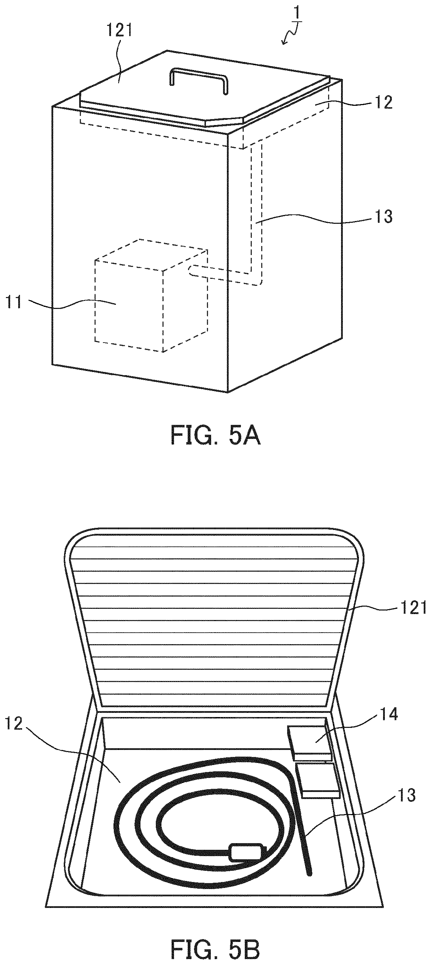

FIG. 5 shows an example of the sterilization apparatus of the present invention. FIG. 5 merely shows an example, and the present invention is not limited to this example. FIG. 5(A) is a perspective view schematically showing a sterilization apparatus 1, and FIG. 5(B) is a perspective view of an instrument housing portion 12 of the sterilization apparatus 1 in a state where a cover 121 is opened.

As shown in FIG. 5(A), the sterilization apparatus 1 includes a reagent housing portion 11, the instrument housing portion 12 provided with the cover 121 that can be opened and closed, a connector 13 that connects the reagent housing portion 11 and the instrument housing portion 12 to each other, and an LED light source 14 is arranged inside the instrument housing portion 12. As shown in FIG. 5(B), the LED light source 14 is arranged on an inner side surface of the instrument housing portion 12, but there is no limitation to this configuration, and the LED light source 14 may be arranged on the cover 121 of the instrument housing portion 12 so as to face the inside of the instrument housing portion 12, for example. The sterilization apparatus 1 can be used as follows, for example. First, the fluid reagent is housed in the reagent housing portion 11 of the sterilization apparatus 1. Then, the cover 112 of the instrument housing portion 12 is opened, an instrument 13 (a photogastroscope is shown as an example in FIG. 5(B)) is housed in the instrument housing portion 12, the cover 121 is closed, and the fluid reagent is introduced from the reagent housing portion 11 via the connector 13 into the instrument housing portion 12. Next, light is emitted from the LED light source 14 toward the fluid reagent introduced into the instrument housing portion 12. As a result, radicals are generated from the radical generation source, and the instrument 13 arranged in the instrument housing portion 12 is sterilized with the radicals. Although a photogastroscope is shown as an example in FIG. 5, there is no limitation thereto, and the sterilization apparatus is applicable to various instruments that need to be sterilized.

### Cancer treatment drug and cancer treatment method

As described above, the cancer treatment drug according to the present invention contains a radical generation source, and cancer cells are treated with radicals generated from the radical generation source.

Also, a cancer treatment method according to the present invention includes a cancer cell treatment step of generating radicals from a radical generation source and treating cancer cells with the radicals.

As shown in an experiment conducted in a reference example, which will be described later, a sterilization effect can be achieved for yeast cells and candida cells through photoirradiation performed in the presence of the radical generation source. In this method, target portions of the yeast cells and the candida cells may be a respiratory chain and a GTP synthesis pathway in mitochondria.

Tissues of animals (e.g., humans) are constituted by cells that belong to eukaryotes, similarly to yeast and candida. Accordingly, the internal structure of the cells of animals is similar to that of eukaryotes such as yeast and candida, and in particular, the mitochondrial respiratory chain, ATP synthase, and the like of the cells of animals are very similar to those of eukaryotes. Therefore, it is supposed that not only cell spores, but also cancer cells can be killed using the present invention.

Cancer cells depend on glycolysis for the production of energy, and suppress the usage rate of the respiratory chain, when compared with normal cells (Warburg's effect). However, mitochondria are present in cancer cells as well, and the respiratory chain functions even though its usage rate is low. Also, it can be thought that cancer cells are very sensitive to ROS (Reactive Oxygen Species) and therefore depend on glycolysis for the production of energy. Accordingly, there is a possibility that cancer cells will be damaged more than normal cells by radicals or ROS generated using the present invention.

Also, the GTP synthesis amount in cancer cells is about twice as much as that in normal cells due to peptide synthesis being actively carried out by ribosomes, and an example in which the GTP synthesis pathway is targeted by an anticancer agent is known. In a case where portions targeted by radicals or ROS generated using the present invention are the respiratory chain and the GTP synthesis pathway in mitochondria as described above, there is a possibility that the radicals or ROS cause great damage to cancer cells, when compared with normal cells.

If there is no difference between normal cells and cancer cells in sensitivity to radicals or ROS generated using the present invention, it is preferable to narrow the area to be irradiated with light from an LED or the like to only cancer cells or cancer tissues and to selectively irradiate only cancer cells or cancer tissues with light, as far as possible. In this case, it is possible to selectively damage cancer cells or cancer tissues while suppressing damage to normal cells.

The document shown below discloses that when yeast (not spores) is irradiated with light from an LED having a wavelength of 455 nm with strong energy (526 J/cm²), the survival rate decreases to one tenth of the survival rate of a case where the irradiation is not performed. According to the document shown below, singlet oxygen is generated as a result of flavin in the cells being irradiated with the light from the LED. In the present invention, the radical generation source is used, and therefore, the energy of photoirradiation can be reduced to a very low energy compared with that in the document shown below, for example. Therefore, it is possible to selectively damage cancer cells or cancer tissues while suppressing damage to normal cells, for example.

405nm and 450nm Photoinactivation of Saccharomyces cerevisiae K. Hoenes, M. Hess, P. Vatter, B. Spellerberg and M. Hessling European Journal of Microbiology and Immunology, 8(4): 142-148 (2018).

The cancer treatment drug of the present invention may be configured such that radicals can be generated when the radical generation source is irradiated with light, for example.

The cancer treatment drug of the present invention may be configured such that the peak wavelength of light used in the photoirradiation is greater than UV wavelengths and 600 nm or less, for example.

The cancer treatment drug of the present invention may be configured such that the peak wavelength of light used in the photoirradiation is 405 to 470 nm, for example.

The cancer treatment drug of the present invention may be configured such that the lower limit of the range of wavelengths included in the light is 380 nm or more, for example.

The cancer treatment drug of the present invention may be configured such that the photoirradiation is performed using an LED, for example.

The cancer treatment drug of the present invention may be configured such that the peak wavelength of the LED is greater than UV wavelengths and 600 nm or less, and a spectral line half width (full width at half maximum) of the LED is 12 to 35 nm.

The cancer treatment drug of the present invention may be configured such that radicals are generated from the radical generation source in the absence of cancer cells, and cancer cells are treated with the radicals, for example.

The cancer treatment drug of the present invention may be configured such that radicals are generated from the radical generation source in the presence of cancer cells, and the cancer cells are treated with the radicals, for example.

The cancer treatment drug of the present invention may be a fluid medicine that contains the radical generation source, for example.

The fluid medicine that is the cancer treatment drug of the present invention, may be a liquid or a gel, for example.

In the cancer treatment drug of the present invention, a solvent in the fluid medicine may be water or a buffer solution, for example.

In the cancer treatment drug of the present invention, the pH of the fluid medicine may be 5 to 10, for example.

In the cancer treatment drug of the present invention, the radical generation source may include at least one selected from the group consisting of halogen ions, hypohalous acid ions, halous acid ions, halogen acid ions, and perhalogen acid ions, for example.

In the cancer treatment drug of the present invention, the halous acid ions may be chlorite ions, for example.

As for the cancer treatment drug of the present invention, the cancer cells may be epithelial carcinoma cells, and the cancer may be epithelial carcinoma, for example.

As for the cancer treatment drug of the present invention, the epithelial carcinoma may be cancer of at least one tissue selected from the group consisting of the urinary system, the reproductive system, the digestive system, the circulatory system, and the respiratory system, for example.

As for the cancer treatment drug of the present invention, the epithelial carcinoma may be at least one cancer of tissue selected from the group consisting of bladder cancer, uterine cancer, cervical cancer, ovarian cancer, carcinoma of a fallopian tube, testis cancer, prostate cancer, colon cancer, small intestinal cancer, duodenal cancer, gastric cancer, esophageal cancer, laryngeal cancer, oral cancer, tongue cancer, pharyngeal cancer, liver cancer, pancreatic cancer, gallbladder cancer, spleen cancer, peritoneal cancer, lung cancer, and eye cancer.

There is no particular limitation on the form of the cancer treatment drug of the present invention, and the cancer treatment drug may be in the form of solid, powder, semisolid, or liquid, for example. When the cancer treatment drug is a solid, the drug may be a tablet or a capsule, for example.

There is no particular limitation on the method for manufacturing the cancer treatment drug of the present invention, and the cancer treatment drug can be manufactured using a method that is the same as or similar to a common method for manufacturing a medicine, for example. Specifically, the cancer treatment drug of the present invention may be manufactured by merely mixing all the components thereof, for example. Alternatively, after mixing all the components, the mixture may be formed into a tablet by being pressed or may be enclosed in a capsule, for example.

The cancer treatment drug of the present invention may optionally contain an optional component other than the radical generation source. As described above, there is no particular limitation on the optional component, and the optional component may be the same as or similar to that contained in a common medicine, for example. In the case where the cancer treatment drug of the present invention is a liquid, for example, examples of the optional component include water, a pH buffer solution, and a physiological saline solution.

The cancer treatment drug of the present invention may further contain, as the optional component, one or more pharmaceutically acceptable additives, for example. That is, for example, a sulfated polysaccharide or a sulfated oligosaccharide may be formulated with one or more pharmaceutically acceptable additives prior to administration. The additives are not particularly limited, and examples thereof include inert substances such as carriers, diluents, flavoring agents, sweeteners, lubricants, solubilizers, suspending agents, binders, tablet disintegrants, and encapsulants. In addition to these, for example, any additive that is commonly used in the field of medicine may be used as appropriate.

In a case where the cancer treatment drug of the present invention is orally administered, the following substances can be used as the additives, for example. As the carrier, for example, lactose, starch, sucrose, glucose, sodium carbonate, mannitol, sorbitol, calcium carbonate, calcium phosphate, calcium sulfate, methyl cellulose, and the like can be used. As the disintegrant, for example, corn flour, starch, methyl cellulose, agar, bentonite, xanthan gum, alginic acid, and the like can be used. As the binder, for example, gelatin, natural sugar, betalactose, corn sweetener, natural and synthetic rubber, gum arabic, tragacanth gum, sodium alginate, carboxymethyl cellulose, polyethylene glycol, wax, and the like can be used. As the lubricant, for example, magnesium stearate, sodium stearate, stearic acid, sodium oleate, sodium benzoate, sodium acetate, salt, talc, and the like can be used.

As for the cancer treatment drug of the present invention, the radical generation source and optionally other optional components may be mixed with a diluent or enclosed in a carrier, for example. The carrier can be in the form of, for example, capsules, sachets, paper, or other containers. The carrier may also serve as a diluent, and may be a solid, a semisolid, or a liquid that acts as a vehicle. The form of the medicine of the present invention is not particularly limited, and it is possible to employ various forms such as tablets, pills, powder, lozenge, elixir, suspension, emulsion, solution, syrup, aerosol, ointment, soft or hard gelatin capsules, suppository, sterilized injection solution, and packaged sterilized powder.

There is no particular limitation on the manner of administration of the cancer treatment drug of the present invention, and the cancer treatment drug can be administered orally or nonorally according to the purpose. In the case where the drug is administered orally, the form of the drug is not particular limited, and can be selected from forms that are ordinarily used by those skilled in the art, such as ordinary and enteric tablets, capsules, pills, powder, granules, elixir, tincture, solvent, suspension, syrup, solid or liquid aerosol, and emulsion. Also, in the case where the drug is administered nonorally, the manner of administration of the drug is not particularly limited, and can be selected from manners that are ordinarily used by those skilled in the art, such as intravenous administration, intraperitoneal administration, subcutaneous administration, intramuscular administration, injection, and infusion.

The dose, administration intervals, and the like of the cancer treatment drug of the present invention are not particularly limited and can be selected as appropriate according to the purpose. Those skilled in the art can select these in view of various factors such as the age, the weight, the sex, and a medical state of a patient, the state of a disease, the route of administration, the level of metabolic and excretory functions of the patient, the form of medicine to be used, a specific oxoacid, oxoacid ions, or a salt of an oxoacid to be administered.

Next, the cancer treatment method according to the present invention includes a cancer cell treatment step of generating radicals from a radical generation source and treating cancer cells with the radicals.

In the cancer treatment method of the present invention, radicals may be generated by irradiating the radical generation source with light in the cancer cell treatment step, for example.

In the cancer treatment method of the present invention, the peak wavelength of light used in the photoirradiation may be greater than UV wavelengths and 600 nm or less, for example.

In the cancer treatment method of the present invention, the peak wavelength of light used in the photoirradiation may be 405 to 470 nm, for example.

In the cancer treatment method of the present invention, the lower limit of the range of wavelengths included in the light may be 380 nm or more, for example.

In the cancer treatment method of the present invention, the photoirradiation may be performed using an LED, for example.

In the cancer treatment method of the present invention, the peak wavelength of the LED may be greater than UV wavelengths and 600 nm or less, and a spectral line half width (full width at half maximum) of the LED may be 12 to 35 nm, for example.

In the cancer treatment method of the present invention, the treatment step may include a step of generating radicals from the radical generation source in the absence of cancer cells, and a step of treating cancer cells with the radicals, for example.

In the cancer treatment method of the present invention, the treatment step may include a step of generating radicals from the radical generation source in the presence of cancer cells and treating the cancer cells with the radicals, for example.

In the cancer treatment method of the present invention, the radicals may be generated from a fluid reagent that contains the radical generation source, for example.

In the cancer treatment method of the present invention, the fluid reagent may be a liquid or a gel, for example.

In the cancer treatment method of the present invention, a solvent in the fluid reagent may be water or a buffer solution, for example.

In the cancer treatment method of the present invention, the pH of the fluid reagent may be 5 to 10, for example.

In the cancer treatment method of the present invention, the radical generation source may include at least one selected from the group consisting of halogen ions, hypohalous acid ions, halous acid ions, halogen acid ions, and perhalogen acid ions, for example.

In the cancer treatment method of the present invention, the halous acid ions may be chlorite ions, for example.

In the cancer treatment method of the present invention, the cancer cells may be epithelial carcinoma cells, and the cancer may be epithelial carcinoma, for example.

In the cancer treatment method of the present invention, the epithelial carcinoma may be cancer of at least one tissue selected from the group consisting of the urinary system, the reproductive system, the digestive system, the circulatory system, and the respiratory system, for example.

In the cancer treatment method of the present invention, the epithelial carcinoma may be at least one cancer of tissue selected from the group consisting of bladder cancer, uterine cancer, cervical cancer, ovarian cancer, carcinoma of a fallopian tube, testis cancer, prostate cancer, colon cancer, small intestinal cancer, duodenal cancer, gastric cancer, esophageal cancer, laryngeal cancer, oral cancer, tongue cancer, pharyngeal cancer, liver cancer, pancreatic cancer, gallbladder cancer, spleen cancer, peritoneal cancer, lung cancer, and eye cancer, for example.

In the cancer treatment method of the present invention, the radical generation source may be the radical generation source contained in the cancer treatment drug of the present invention, and the cancer treatment method may further include an administration step of administering the cancer treatment drug to a patient, for example.

According to the present invention, it is possible to further provide a method of using the treatment drug of the present invention in cancer treatment.

The method of using the cancer treatment drug of the present invention may include an administration step of administering the cancer treatment drug of the present invention to a patient, for example.

According to the present invention, it is possible to further provide a method of using the radical generation source to manufacture the cancer treatment drug of the present invention. As described above, there is no particular limitation on the method for manufacturing the cancer treatment drug of the present invention.

Examples of the patient to which the cancer treatment drug is administered or that is treated using the cancer treatment method of the present invention include a human and a non-human animal excluding humans, for example. Examples of the non-human animal include a mouse, a rat, a rabbit, a monkey, a dog, and cattle.

### Examples

The following describes examples of the present invention. However, the present invention is not limited to the following examples.

### Example 1

Spores of Bacillus subtilis were sterilized using the method of the present invention, and a reduction in the number of spores was confirmed.

### (1) Radical concentration check

A sodium chlorite solution (a chlorite ion standard solution, FUJIFILM Wako Pure Chemical Corporation) was used as the radical generation source. The molar concentration of sodium chlorite was set to 14.5 mmol/L, and reagents of which the pH was adjusted to 7.0, 7.5, and 8.0 were prepared by adding a 20 mmol/L potassium phosphate buffer solution. 1 mL of each of the reagents was placed in a disposable cell UV (product name: Two-face transparent semimicro #1941, Nikko Hansen & CO., LTD.), and was irradiated with light via a side surface of the cell using an LED. A commercially available LED (product name: M455L4, Thorlabs Japan Inc.) having a peak wavelength of 455 nm and a spectral line half width of 15 nm was used as the LED, a distance between the side surface of the cell irradiated with light and a light emitting surface of the LED was set to 0.3 cm, and the irradiation was performed with an irradiance of 157 mW/cm² for 40 minutes. The radiant energy under the above condition was calculated as follows: 0.157(W/cm²)×40(minutes)×60(seconds)=377(J/cm²).

Then, absorption in the range from 450 nm to 240 nm was measured with respect to the reagent in the cell using a double optical path spectrophotometer (product name: UV-2400PC, SHIMADZU CORPORATION) to determine the molar concentration of chlorite ions and the molar concentration of chlorine dioxide radicals. The results are shown in Table 1 below. Table 1 below shows results of the concentrations measured six times (n=6) under each pH condition. As shown in Table 1, it was confirmed that chlorine dioxide radicals were generated through photoirradiation of the reagents performed using the LED. The closer the pH of a reagent was to being acidic, the greater the amount of generated chlorine dioxide radicals was.

**Table 1**

| pH | Photoirradiation | Chlorine dioxide radicals µmol/L | Chlorite ions mmol/L |
|---|---|---|---|
| 7.0 | Not performed | 0 | 14.5 |
| 7.0 | Performed | 700-800 | 7.5-8.5 |
| 7.5 | Performed | 500-600 | 9.5-10.5 |
| 8.0 | Performed | 350-450 | 11.0-12.0 |

### (2) Spore sterilization treatment

Since the generation of radicals through photoirradiation performed under the above-described predetermined conditions was confirmed, a spore sterilization effect of the radicals was checked.

Bacillus subtilis was scattered over a nutrient agar medium and incubated at 37°C for 1 week. Colonies were scraped off from the agar medium and washed by being suspended in a physiological saline solution, and Bacillus subtilis collected through centrifugal separation was taken to be cell spores. The cell spores were suspended in a physiological saline solution at a concentration of about 6×10⁶ spores/mL. The suspension of cell spores was prepared so as to have the target concentration of cell spores by being diluted with purified water as appropriate.

On the other hand, a reagent having a pH of 7.0 was prepared and irradiated with light using the LED in the same manner as that described above in (1) to generate radicals. The reagent (chlorine dioxide radical concentration: 600 µmol/L) in which radicals were generated was diluted with a 10 mmol/L potassium phosphate buffer solution (pH: 7.0) as appropriate to prepare samples containing radicals at predetermined concentrations. 0.5 mL of each of the samples was mixed with 0.5 mL of the suspension of cell spores described above, and the liquid mixture was let to stand at room temperature for 30 minutes. Thereafter, the liquid mixture was scattered over brain heart agar, incubation was performed at 37°C for 12 hours, and then the number of colonies was counted.

As control 1, a reagent having a pH of 7.0 that was not irradiated with light was added to the suspension of cell spores and was left to stand, and incubation was performed as described above, and then the number of colonies was counted. As control 2, the suspension of cell spores to which the reagent was not added was irradiated with light for 30 minutes under the irradiation conditions described above in (1) using the LED, thereafter incubation was performed as described above, and then the number of colonies was counted.

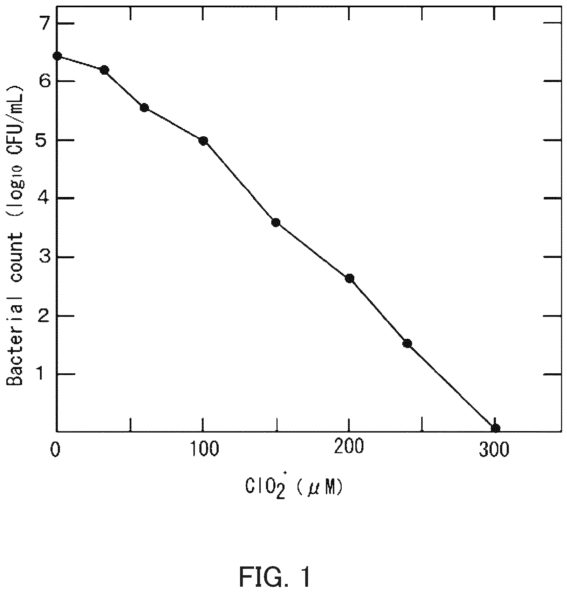

The results are shown in FIG. 1. In FIG. 1, the vertical axis indicates the number of grown spores, and the horizontal axis indicates the chlorine dioxide radical concentration in the spore suspension scattered over the agar medium. A reduction in the number of spores was not confirmed in controls 1 and 2, whereas it was confirmed that chlorine dioxide radicals were generated through the photoirradiation in this Example, and cell spores were killed according to the chlorine dioxide radical concentration as shown in FIG. 1. When the chlorine dioxide radical concentration was 300 µmol/L, the number of spores decreased to 10⁻⁶ or less, and it was also confirmed that the sterilization method was very effective. Also, in this Example, the wavelength of light used in the photoirradiation did not include ultraviolet rays that affect human bodies, for example, and the reagent and generated radicals were used at concentrations that were safe for human bodies, and therefore, it can be said that killing of spores, which has conventionally been difficult, can be realized very safely.

### Example 2

A sodium chlorite solution (a chlorite ion standard solution, FUJIFILM Wako Pure Chemical Corporation) was used as the radical generation source. Reagents having molar concentrations of sodium chlorite of 14.5 mmol/L, 7.25 mmol/L, and 3.63mmol/L were prepared using a 20 mmol/L potassium phosphate buffer solution (pH: 7.0). 1 mL of each of the reagents was placed in a disposable cell UV (product name: Two-face transparent semimicro #1941, Nikko Hansen & CO., LTD.), and was irradiated with light via a side surface of the cell using an LED in the same manner as in Example 1 (1) described above. The photoirradiation was performed similarly to Example 1 described above, unless otherwise specified. That is, the same commercially available LED (product name: M455L4, Thorlabs Japan Inc.) as that used in Example 1, which had a peak wavelength of 455 nm and a spectral line half width of 15 nm, was used, the distance between the side surface of the cell irradiated with light and the light emitting surface of the LED was set to 0.3 cm, and the irradiation was performed with an irradiance of 81.5 mW/cm² or 157 mW/cm². Then, an absorption spectrum was measured over time from the start of irradiation using the double optical path spectrophotometer (product name: UV-2400PC, SHIMADZU CORPORATION), and the molar concentration of chlorite ions (ClO₂⁻) was calculated from an absorbance at the minimum absorption of the wavelength of 260 nm, and the molar concentration of chlorine dioxide radicals (ClO₂^{·}) was calculated from an absorbance at the maximum absorption of the wavelength of 359 nm.

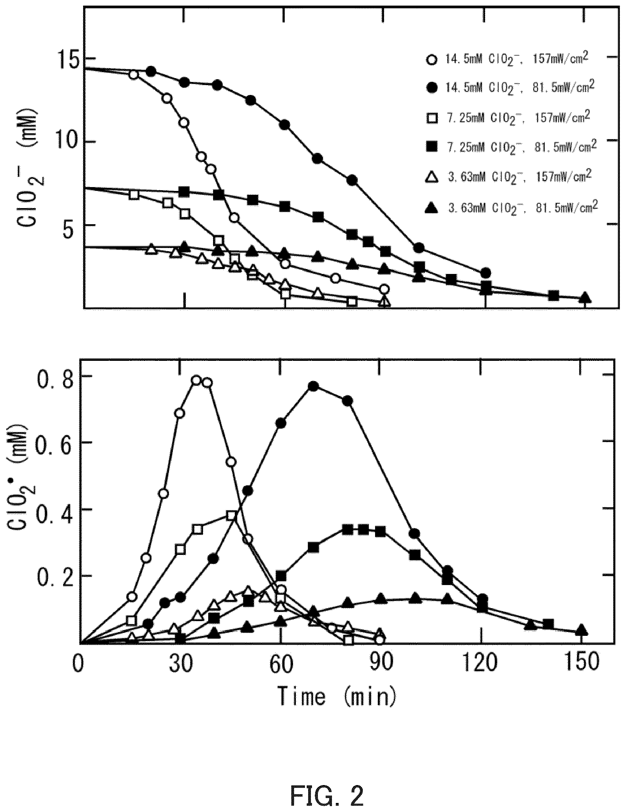

The results are shown in FIG. 2. In the upper graph shown in FIG. 2, the horizontal axis indicates the irradiation time (graduations are the same as those in the lower graph) and the vertical axis indicates the molar concentration of ClO₂⁻, and in the lower graph shown in FIG. 2, the horizontal axis indicates the irradiation time and the vertical axis indicates the molar concentration of ClO₂^{·}. In both graphs shown in FIG. 2, black plotted points show results obtained with the irradiance of 81.5 mW/cm², and white plotted points show results obtained with the irradiance of 157 mW/cm².

As shown in the lower graph in FIG. 2, it was found that when the irradiance in the LED irradiation was doubled, the time it took for the molar concentration of ClO₂^{·} to reach the same maximum peak decreased by 1/2. Also, as shown in the upper graph in FIG. 2, it was found that when the ClO₂⁻ concentration in the reagent was doubled, the height of the maximum peak increased about twofold. These results show that it is possible to adjust the concentration of chlorine dioxide radicals and the irradiation time with which the generation of chlorine dioxide radicals becomes greatest by setting the concentration of chlorite ions in the reagent and the irradiance as appropriate, for example. Also, it was confirmed that when photoirradiation was performed in the same manner except that an LED having a peak wavelength of 405 nm was used, the time it took for the concentration of chlorine dioxide radicals to reach a maximum peak decreased to about 1/10 to 1/15 of the time it took for the concentration to reach a maximum peak when photoirradiation was performed using the above-described LED. This result shows that it is also possible to adjust the irradiation time with which the generation of chlorine dioxide radicals becomes greatest by adjusting the peak wavelength of the light source used in the photoirradiation.

It is supposed that the following reaction occurs when a reagent containing chlorite ions is irradiated with light under the above-described conditions, but the following reaction by no means limits the present invention.

[C1] (about 5%, the remaining 95% is triplet oxygen) Cl· + ClO₂⁻ → Cl⁻ + ClO₂· ¹O₂ + ClO₂⁻ → O₂·⁻ + ClO₂· 2O₂·⁻ + 2H⁻ → H₂O₂ + O₂ (disproportionation reaction)

Furthermore, as shown in FIG. 2, in this Example, it was confirmed that rather than the ClO₂⁻ concentration simply decreasing, the ClO₂⁻ concentration decreased at about the same rate after the ClO₂^{·} concentration increased. The ClO₂^{·} concentration decreased by about half in 2 hours when photoirradiation was not performed. It is thought that this is due to ClO₂^{·} diffusing from a water layer to an air layer. Also, as shown in the above chemical reaction formula [C1], it is thought that ClO₂^{·} changed into H₂O₂ via O₂⁻. As described above, in this Example, it was confirmed that only H₂O₂ finally remained at a low concentration with almost no ClO₂⁻, ClO₂^{·}, and singlet oxygen (having an extremely short lifetime) remaining. When compared with ClO₂^{·}, H₂O₂ has extremely low toxicity to living bodies, and its toxicity to cells and living bodies is extremely low at a concentration of about 800 µM (0.0027 W/V%, 27 ppm), which is an estimated maximum concentration of H₂O₂ to be generated.

### Example 3

The generation of singlet oxygen through LED irradiation of a radical generation source was confirmed indirectly.

A sodium chlorite solution (a chlorite standard solution, FUJIFILM Wako Pure Chemical Corporation) was used as the radical generation source. Reagents having molar concentrations of sodium chlorite of 14.5 mmol/L, 7.25 mmol/L, and 3.63mmol/L were prepared using a 20 mmol/L potassium phosphate buffer solution (pH: 7.0). Then, the same spores as those used in Example 1 described above were added to each of the reagents such that 1×10⁶ to 1×10⁷ spores were contained in each reagent. Next, 300 µL of each of the reagents having the respective concentrations to which the spores were added was placed in an oxygen measurement device provided with an oxygen electrode. The oxygen electrode (product name: Biological dissolved oxygen monitor, digital 1ch OXY018A) and a 2.5 mL Perspex electrode (OXY042A), Rank Brothers Ltd.) were disposed in the oxygen measurement device. An LED was attached to an upper portion of the oxygen electrode, and the reagent was irradiated with light. The commercially available LED (product name: M455L4, Thorlabs Japan Inc.) having a peak wavelength of 455 nm and a spectral line half width of 15 nm was used as the LED, a distance between the surface of the reagent irradiated with light and a light emitting surface of the LED was set to 3 cm, and the irradiation was performed with an irradiance of 300 mW/cm². Then, an absorption spectrum was measured over time from the start of irradiation using the double optical path spectrophotometer (product name: UV-2400PC, SHIMADZU CORPORATION), and the molar concentration of chlorine dioxide radicals (ClO₂^{·}) was calculated from an absorbance at the maximum absorption of the wavelength of 359 nm. Also, the molar concentration of oxygen was measured using the oxygen measurement device.

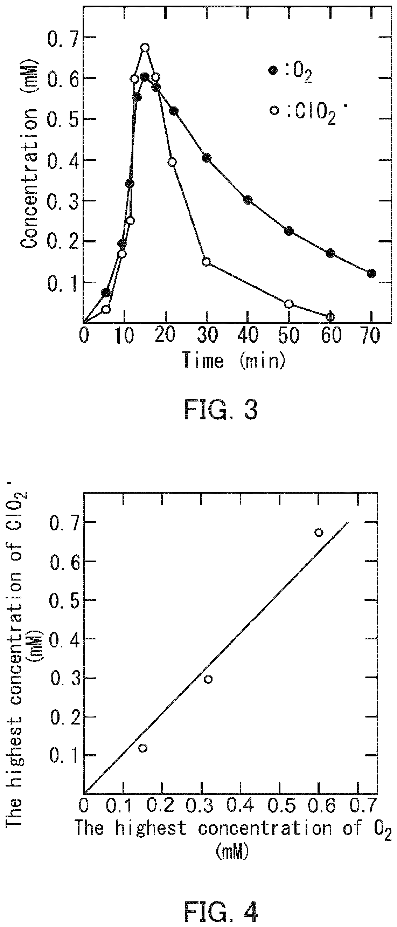

FIG. 3 shows changes in the chlorine dioxide radical concentration and the oxygen concentration over time with respect to the reagent having a sodium chlorite molar concentration of 14.5 mmol/L. In FIG. 3, the vertical axis indicates molar concentrations of oxygen and chlorine dioxide radicals, and the horizontal axis indicates the irradiation time. FIG. 4 shows results of maximum values of the molar concentrations of oxygen and chlorine dioxide radicals with respect to the reagents having sodium chlorite molar concentrations of 14.5 mmol/L, 7.25 mmol/L, and 3.63 mmol/L. In FIG. 4, the vertical axis indicates the maximum value of the molar concentration of chlorine dioxide radicals and the horizontal axis indicates the maximum value of the molar concentration of oxygen. In FIG. 4, the left plotted point shows the result of the reagent having a sodium chlorite molar concentration of 14.5 mmol/L, the middle plotted point shows the result of the reagent having a sodium chlorite molar concentration of 7.25 mmol/L, and the right plotted point shows the result of the reagent having a sodium chlorite molar concentration of 3.63 mmol/L.

The following was found from the results shown in FIGS. 3 and 4. As shown in FIG. 3, the ClO₂^{·} concentration reached the highest value that was about 0.7 mmol/L in 20 minutes from the start of irradiation performed using the LED having a peak wavelength of 455 nm, and the oxygen concentration reached about 0.6 mmol/L at approximately the same time. From this result, it was found that a sufficiently large amount of ClO₂^{·} for sterilizing spores was generated with an irradiation time of 20 minutes. Also, it was found that there is a proportional relationship between the highest concentration of ClO₂^{·} and the highest concentration of oxygen as shown in FIG. 4. Furthermore, the generation of a sufficiently large amount of oxygen was confirmed using the oxygen electrode, and it can be said that this result indirectly proves the generation of singlet oxygen. When the spores (1×10⁶/300 µL) were irradiated with light for 30 minutes using the LED having a peak wavelength of 455 nm (irradiation condition: 300 mW/cm²) in the presence of 3.63 mmol/L of sodium chlorite (20 mmol/L phosphate buffer solution having a pH of 7.0), the number of spores decreased to 1×100/300 µL or less.

### Example 4

Yeast cells and candida cells were sterilized by being irradiated with light in the presence of sodium chlorite, and a reduction in the number of colonies was confirmed.

### (1) Preparation of yeast cells

Yeast was inoculated into a modified YPE liquid medium (2% of polypeptone, 1% of yeast extract, 2% of ethanol, and 0.2% of glucose) and was incubated at 30°C for 1 day. A bacterial liquid was collected from the liquid medium and washed by being suspended in purified water, and yeast cells collected through centrifugal separation were taken as a specimen. The cells were suspended in a 100 mmol/L potassium phosphate buffer solution (pH: 7.0) at a concentration of about 5×10⁸ cells/mL. Also, the suspension of cells was diluted with a 100 mmol/L potassium phosphate buffer solution as appropriate so as to have a target concentration of cells.

### (2) Preparation of reagent (aqueous solution) containing sodium chlorite

A sodium chlorite solution (a chlorite ion standard solution, FUJIFILM Wako Pure Chemical Corporation) was used as the radical generation source. The molar concentration of sodium chlorite was set to 7.25 mmol/L, and a reagent having a pH of 7.0 was prepared by adding a 20 mmol/L potassium phosphate buffer solution.

### (3) Photoirradiation of sodium chlorite aqueous solution

The reagent prepared as described above in (2) was irradiated with light having a peak wavelength of 455 nm and a spectral line half width of 15 nm using an LED in the same manner as that described in Example 1 "(1) Radical concentration check", except that the molar concentration of sodium chlorite was 7.25 mmol/L. The irradiation was performed with an irradiance of 157 mW/cm² for 40 minutes. After the irradiation, absorption in the range from 450 nm to 240 nm was measured with respect to the reagent in the cell using the double optical path spectrophotometer (product name: UV-2400PC, SHIMADZU CORPORATION) to determine the molar concentration of chlorite ions and the molar concentration of chlorine dioxide radicals, and it was found that the molar concentration of chlorite ions was 350 mmol/L and the molar concentration of chlorine dioxide radicals was 375 µmol/L. Thus, a reagent containing chlorine dioxide radicals was prepared.

### (4) Yeast sterilization treatment

Yeast sterilization treatment was performed using two methods described in the following Experiment 1 and Experiment 2.

### (4-1) Experiment 1

The reagent containing chlorine dioxide radicals prepared as described above in (3) was diluted with a 10 mmol/L potassium phosphate buffer solution (pH: 7.0) to prepare a sample containing chlorine dioxide radicals at a concentration of 20 µmol/L. On the other hand, the suspension of cells prepared in (1) was diluted with a 10 mmol/L potassium phosphate buffer solution (pH: 7.0) such that the concentration of cells was about 5×10⁸ cells/mL. Next, 0.5 mL of the sample containing chlorine dioxide radicals was mixed with 0.5 mL of the suspension of cell spores, and the liquid mixture was left to stand at room temperature for 30 minutes. Thereafter, the liquid mixture was scattered over brain heart agar and incubated at 30°C for 1 day, and then the number of colonies was counted. As a result, it was confirmed that the concentration of cells was about 2.5×10⁷ cells/mL and the survival rate decreased to about one tenth of the survival rate of a case where the above treatment was not performed.

### (4-2) Experiment 2

A sodium chlorite solution (a chlorite ion standard solution, FUJIFILM Wako Pure Chemical Corporation) was used as the radical generation source. A reagent having a sodium chlorite molar concentration of 3.75 mmol/L was prepared using a 20 mmol/L potassium phosphate buffer solution (pH: 7.0). Then, similarly to Experiment 1 described above, 0.5 mL of a yeast solution having a yeast cell concentration of about 5×10⁸ cells/mL was added to 0.5 mL of the reagent, and 1 mL of the resultant solution was placed in a disposable cell UV (product name: Two-face transparent semimicro #1941, Nikko Hansen & CO., LTD.), and was irradiated with light using an LED via a side surface of the cell. The photoirradiation was performed similarly to Experiment 1 described above, unless otherwise specified. That is, the same commercially available LED (product name: M455L4, Thorlabs Japan Inc.) as that used in Experiment 1, which had a peak wavelength of 455 nm and a spectral line half width of 15 nm, was used, the distance between the side surface of the cell irradiated with light and the light emitting surface of the LED was set to 0.3 cm, and the irradiation was performed with an irradiance of 21 mW/cm² for 30 minutes at 25°C. The radiant energy under the above condition was calculated as 37.8 J/cm². The solution after the treatment was scattered over brain heart agar after being diluted as appropriate, and incubated at 30°C for 1 day, and then the number of colonies was counted. As a result, it was confirmed that the concentration of cells was about 2.5×10⁷ cells/mL and the survival rate decreased to about one tenth of the survival rate of a case where the above treatment was not performed.

Cells were incubated in the same manner as that described in (1) to (4), except that Candida albicans was used instead of yeast, and it was confirmed that the survival rate of the cells similarly decreased to about one tenth of the survival rate of a case where the treatment was not performed.

Furthermore, a reduction in the electron transfer activity of the respiratory chain was observed in yeast cells and candida cells in proportion to the reduction in the survival rate. Accordingly, it is supposed that the respiratory chain in mitochondria was one target portion of the yeast cells and the candida cells treated with chlorine dioxide radicals. It is thought that when the respiratory chain is damaged, ROS (Reactive Oxygen Species) are generated, which leads to apoptosis and the death of cells.

### (5) Control experiment

When yeast cells or candida cells were left to stand at 25°C for 1 hour in the same manner as that described above in (1) to (4), except that the sodium chlorite aqueous solution was not irradiated with light in (3), the survival rate did not decrease. Also, when a suspension that was prepared as described above in "(4) Spore sterilization treatment" and had a cell concentration of about 2.5×10⁸ cells/mL was irradiated with light having a peak wavelength of 455 nm and a spectral line half width of 15 nm with an irradiance of 37.8 J/cm² in the same manner as that described above in "(3) Photoirradiation of sodium chlorite aqueous solution", without the sample containing chlorine dioxide radicals being mixed with the suspension, the survival rate of yeast cells or candida cells did not decrease.

Based on this result, it is thought that chlorine dioxide radicals (ClO₂^{·}) were not generated when only the sodium chlorite aqueous solution was used or only photoirradiation was performed, but when the sodium chlorite aqueous solution was used and photoirradiation was performed, chlorine dioxide radicals were generated and the effect of sterilizing cells was achieved. Also, when the sodium chlorite aqueous solution was used and photoirradiation was performed, singlet oxygen may have been generated in addition to chlorine dioxide radicals, and the effect of sterilizing cells may have been achieved as a synergistic effect of chlorine dioxide radicals and singlet oxygen.

### (6) Reference experiment

Treatment was performed in the same manner as that described in (1) to (4), except that a 2.90 mmol/L aqueous solution of cGMP (cyclic guanosine monophosphate) was used instead of the suspension of cells. As a result, it was found that a substance that hindered the growth of yeast and candida was generated. The generated substance may be a compound that hinders the GTP synthesis pathway, when making a presumption based on the structure of cGMP used as the raw material. Accordingly, in addition to the respiratory chain in mitochondria, the GTP synthesis pathway may be one target portion of yeast cells and candida cells treated with chlorine dioxide radicals in this Example.

### Example 5

Cancer cells were treated using the method of the present invention, and a reduction in the number of cancer cells was confirmed.

HT-29 cells (human colon adenocarcinoma cells) were inoculated into each well of a cell incubation microplate (subjected to surface treatment) having 96 wells and grown in a CO₂ incubator at 37°C in the presence of 5% of CO₂ until the number of cells reached about 2000 cells/well. 90 µL of McCoy's 5a Medium (the name of a product manufactured by FUJIFILM Wako Pure Chemical Corporation) was used as the cell culture medium. Next, after each treatment described below in (1) to (6) was performed, the medium was replaced with 90 µL of McCoy's 5a Medium and the cells were incubated in the CO₂ incubator for 2 days under the above conditions. 10 µL of a WST-8 solution (solution in a product "WST-8 Cell Proliferation Kit", Funakoshi Co., Ltd.) was added to measure the number of living cells, then incubation was performed in the CO₂ incubator (37°C) for 5 hours, and thereafter an absorbance of 450 nm was measured using a microplate reader. The percentage of the absorbance of each sample relative to an absorbance when treatment (1), which was a control experiment, was performed was calculated. The absorbance when treatment (1) was performed was almost the same as the absorbance of untreated cells.

The name of substance "WST-8" in the WST-8 solution is 2-(2-methoxy-4-nitrophenyl)-3-(4-nitrophenyl)-5-(2,4-disulfophenyl)-2H-tetrazolium monosodium salt.

Conditions of treatments (1) to (6) and percentages of absorbances of samples obtained through the treatments were as follows. The amount by which the percentage of an absorbance decreased from 100% is presumed to be substantially equal to a death rate (%) of cancer cells.
(1) The medium was replaced with 100 µL of PBS (pH: 7.0)^{∗∗} and left to stand at room temperature for 30 minutes. ------ Absorbance percentage: 100%
(2) The medium was replaced with 100 µL of PBS (pH: 7.0) and irradiated with light (radiant energy: 72 J/cm²) at room temperature for 30 minutes using an LED (product name: M455L3, Thorlabs Japan Inc., wavelength: 455 nm, irradiance: 40 mW/cm²). ------ Absorbance percentage: 96%
(3) The medium was replaced with 100 µL of PBS (pH: 7.0) containing 1.4 mM of NaClO₂ and left to stand at room temperature for 30 minutes. ------ Absorbance percentage: 97%
(4) The medium was replaced with 100 µL of PBS (pH: 7.0) containing 1.4 mM of NaClO₂ and irradiated with light (radiant energy: 72 J/cm²) at room temperature for 30 minutes using an LED (product name: M455L3, Thorlabs Japan Inc., wavelength: 455 nm, irradiance: 40 mW/cm²).--- Absorbance percentage: 11%
(5) The medium was replaced with 100 µL of PBS (pH: 7.0) containing 2.8 mM of NaClO₂ and left to stand at room temperature for 30 minutes. ------ Absorbance percentage: 97%
(6) The medium was replaced with 100 µL of PBS (pH: 7.0) containing 2.8 mM of NaClO₂ and irradiated with light (radiant energy: 72 J/cm²) at room temperature for 30 minutes using an LED (product name: M455L3, Thorlabs Japan Inc., wavelength: 455 nm, irradiance: 40 mW/cm²).--- Absorbance percentage: 6%
^{∗∗}: A phosphate-buffered saline whose pH was adjusted to 7.0 and osmotic pressure was adjusted to be the same as that of a standard phosphate-buffered saline having a pH of 7.5 was used. Specifically, PBS (pH: 7.0) had the following composition: NaCl: 140 mM, KCl: 0.43 mM, KH₂PO₄:3.72 mM, Na₂HPO₄: 5.81 mM.

In this Example, it was confirmed that when cancer cells were irradiated with visible light having a wavelength of 455 nm with a radiant energy of 72 J/cm² in the presence of NaClO₂ solution, about 89% (1.4 mM NaClO₂) and 94% (2.8 mM NaClO₂) of the cancer cells were killed (see the above treatments (4) and (6)).

As described above, in this Example, cancer cells were treated using the method of the present invention, and a reduction in the number of cancer cells was confirmed.

As described above, human tissues are constituted by cells that belong to eukaryotes, similarly to yeast and candida. Accordingly, the internal structure of human cells is similar to that of eukaryotes such as yeast and candida, and in particular, the mitochondrial respiratory chain, ATP synthase, and the like of human cells are very similar to those of eukaryotes. It is thought that, similarly to Example 4, in this Example, chlorine dioxide radicals and singlet oxygen were quantitatively generated as a result of chlorite ions and violet LED (455 nm) being used in combination, and human cancer cells were killed.

Although the present invention has been described with reference to the embodiment and Examples, the present invention is not limited to the above embodiment and Examples. Various changes that may become apparent to those skilled in the art may be made in the configuration and specifics of the present invention without departing from the scope of the present invention.

### Industrial Applicability

According to the present invention, it is possible to generate radicals from a radical generation source by merely performing photoirradiation using light whose peak wavelength is greater than UV wavelengths and 600 nm or less. Accordingly, for example, spores can be sterilized with the generated radicals. Also, the conditions of the photoirradiation are not conditions that are harsh on the environment, living bodies, and the like, and therefore, spores can be sterilized in a highly safe manner for the environment or an instrument to be treated, an operator, or the like.

This application claims priority from Japanese Patent Application No. 2019-181758 filed on October 1, 2019 and Japanese Patent Application No. 2020-089254 filed on May 21, 2020, the entire content of which is incorporated herein by reference.

## Claims

1. A production method for producing radicals, comprising:
a generation step of generating radicals through photoirradiation of a radical generation source,
wherein a peak wavelength of light used in the photoirradiation is greater than UV wavelengths and 600 nm or less.

2. The production method according to claim 1,
wherein the peak wavelength is 405 to 470 nm.

3. The production method according to claim 1 or 2,
wherein the lower limit of the range of wavelengths included in the light is 380 nm or more.

4. The production method according to any one of claims 1 to 3,
wherein the photoirradiation is performed using an LED.

5. The production method according to claim 4,
wherein a peak wavelength of the LED is greater than UV wavelengths and 600 nm or less, and a spectral line half width (full width at half maximum) of the LED is 12 to 35 nm.

6. The production method according to any one of claims 1 to 5,
wherein the photoirradiation of the radical generation source is photoirradiation of a fluid reagent that contains the radical generation source.

7. The production method according to claim 6,
wherein the fluid reagent is a liquid or a gel.

8. The production method according to claim 6 or 7,
wherein a solvent in the fluid reagent is water or a buffer solution.

9. The production method according to any one of claims 6 to 8,
wherein the pH of the fluid reagent is 5 to 10.

10. The production method according to any one of claims 1 to 9,
wherein the radical generation source includes at least one selected from the group consisting of halogen ions, hypohalous acid ions, halous acid ions, halogen acid ions, and perhalogen acid ions.

11. The production method according to claim 10,
wherein the halous acid ions are chlorite ions.

12. A sterilization method for sterilizing spores, comprising:
a treatment step of generating radicals through photoirradiation of a radical generation source and treating spores with the radicals,
wherein a peak wavelength of light used in the photoirradiation is greater than UV wavelengths and 600 nm or less.

13. The sterilization method according to claim 12,
wherein the peak wavelength is 405 to 470 nm.

14. The sterilization method according to claim 12 or 13,
wherein the lower limit of the range of wavelengths included in the light is 380 nm or more.

15. The sterilization method according to any one of claims 12 to 14,
wherein the treatment step includes:
a step of generating radicals through photoirradiation of the radical generation source in the absence of spores; and
a step of treating spores with the radicals.

16. The sterilization method according to any one of claims 12 to 14,
wherein the treatment step includes a step of generating radicals through photoirradiation of the radical generation source in the presence of spores and treating the spores with the radicals.

17. The sterilization method according to any one of claims 12 to 16,
wherein the photoirradiation is performed using an LED.

18. The sterilization method according to claim 17,
wherein a peak wavelength of the LED is greater than UV wavelengths and 600 nm or less, and a spectral line half width (full width at half maximum) of the LED is 12 to 35 nm.

19. The sterilization method according to any one of claims 12 to 18,
wherein the photoirradiation of the radical generation source is photoirradiation of a fluid reagent that contains the radical generation source.

20. The sterilization method according to claim 19,
wherein the fluid reagent is a liquid or a gel.

21. The sterilization method according to claim 19 or 20,
wherein a solvent in the fluid reagent is water or a buffer solution.

22. The sterilization method according to any one of claims 19 to 21,
wherein the pH of the fluid reagent is 5 to 10.

23. The sterilization method according to any one of claims 12 to 22,
wherein the radical generation source includes at least one selected from the group consisting of halogen ions, hypohalous acid ions, halous acid ions, halogen acid ions, and perhalogen acid ions.

24. The sterilization method according to claim 23,
wherein the halous acid ions are chlorite ions.

25. The sterilization method according to any one of claims 12 to 24,
wherein the spores are spores of the genus Bacillus or the genus Clostridium.

26. A sterilization apparatus for sterilizing an instrument, comprising:
a reagent housing portion;
an instrument housing portion;
a connector; and
a photoirradiation portion,
wherein the instrument housing portion and the reagent housing portion are connected to the connector such that a reagent housed in the reagent housing portion is introduced into the instrument housing portion,
the reagent housing portion is configured to house a reagent that contains a radical generation source,
the instrument housing portion is configured to house an instrument to be sterilized,
the photoirradiation portion includes a light source configured to emit light toward at least one of the reagent housing portion and the instrument housing portion, and
a peak wavelength of light emitted from the light source is greater than UV wavelengths and 600 nm or less.

27. The sterilization apparatus according to claim 26,
wherein the reagent is a fluid reagent and contains the radical generation source and a fluid solvent.

28. The sterilization apparatus according to claim 26 or 27,
wherein the reagent housing portion is a portion that houses the fluid solvent, and
when the radical generation source is added to the fluid solvent housed in the reagent housing portion, the fluid reagent containing the radical generation source is produced.

29. A cancer treatment drug comprising:
a radical generation source,
wherein cancer cells are treated with radicals generated from the radical generation source.

30. The cancer treatment drug according to claim 29,
wherein radicals can be generated through photoirradiation of the radical generation source.

31. The cancer treatment drug according to claim 30,
wherein a peak wavelength of light used in the photoirradiation is greater than UV wavelengths and 600 nm or less.

32. The cancer treatment drug according to claim 31,
wherein the peak wavelength is 405 to 470 nm.

33. The cancer treatment drug according to claim 31 or 32,
wherein the lower limit of the range of wavelengths included in the light is 380 nm or more.

34. The cancer treatment drug according to any one of claims 30 to 33,
wherein the photoirradiation is performed using an LED.

35. The cancer treatment drug according to claim 34,
wherein a peak wavelength of the LED is greater than UV wavelengths and 600 nm or less, and a spectral line half width (full width at half maximum) of the LED is 12 to 35 nm.

36. The cancer treatment drug according to any one of claims 29 to 35,
wherein radicals are generated from the radical generation source in the absence of cancer cells, and cancer cells are treated with the radicals.

37. The cancer treatment drug according to any one of claims 29 to 35,
wherein radicals are generated from the radical generation source in the presence of cancer cells, and the cancer cells are treated with the radicals.

38. The cancer treatment drug according to any one of claims 29 to 37, which is a fluid medicine containing the radical generation source.

39. The cancer treatment drug according to claim 38,
wherein the fluid medicine is a liquid or a gel.

40. The cancer treatment drug according to claim 38 or 39,
wherein a solvent in the fluid medicine is water or a buffer solution.

41. The cancer treatment drug according to any one of claims 38 to 40,
wherein the pH of the fluid medicine is 5 to 10.

42. The cancer treatment drug according to any one of claims 29 to 41,
wherein the radical generation source includes at least one selected from the group consisting of halogen ions, hypohalous acid ions, halous acid ions, halogen acid ions, and perhalogen acid ions.

43. The cancer treatment drug according to claim 42,
wherein the halous acid ions are chlorite ions.

44. The cancer treatment drug according to any one of claims 29 to 43,
wherein the cancer cells are epithelial carcinoma cells, and the cancer is epithelial carcinoma.

45. The cancer treatment drug according to claim 44,
wherein the epithelial carcinoma is cancer of at least one tissue selected from the group consisting of the urinary system, the reproductive system, the digestive system, the circulatory system, and the respiratory system.

46. The cancer treatment drug according to claim 44 or 45,
wherein the epithelial carcinoma is at least one cancer of tissue selected from the group consisting of bladder cancer, uterine cancer, cervical cancer, ovarian cancer, carcinoma of a fallopian tube, testis cancer, prostate cancer, colon cancer, small intestinal cancer, duodenal cancer, gastric cancer, esophageal cancer, laryngeal cancer, oral cancer, tongue cancer, pharyngeal cancer, liver cancer, pancreatic cancer, gallbladder cancer, spleen cancer, peritoneal cancer, lung cancer, and eye cancer.

47. A cancer treatment method comprising:
a cancer cell treatment step of generating radicals from a radical generation source and treating cancer cells with the radicals.

48. The cancer treatment method according to claim 47,
wherein radicals are generated through photoirradiation of the radical generation source in the cancer cell treatment step.

49. The cancer treatment method according to claim 48,
wherein a peak wavelength of light used in the photoirradiation is greater than UV wavelengths and 600 nm or less.

50. The cancer treatment method according to claim 49,
wherein the peak wavelength is 405 to 470 nm.

51. The cancer treatment method according to claim 49 or 50,
wherein the lower limit of the range of wavelengths included in the light is 380 nm or more.

52. The cancer treatment method according to any one of claims 48 to 51,
wherein the photoirradiation is performed using an LED.

53. The cancer treatment method according to claim 52,
wherein a peak wavelength of the LED is greater than UV wavelengths and 600 nm or less, and a spectral line half width (full width at half maximum) of the LED is 12 to 35 nm.

54. The cancer treatment method according to any one of claims 47 to 53,
wherein the treatment step includes:
a step of generating radicals from the radical generation source in the absence of cancer cells; and
a step of treating cancer cells with the radicals.

55. The cancer treatment method according to any one of claims 47 to 53,
wherein the treatment step includes a step of generating radicals from the radical generation source in the presence of cancer cells, and treating the cancer cells with the radicals.

56. The cancer treatment method according to any one of claims 47 to 55,
wherein the radicals are generated from a fluid reagent that contains the radical generation source.

57. The cancer treatment method according to claim 56,
wherein the fluid reagent is a liquid or a gel.

58. The cancer treatment method according to claim 56 or 57,
wherein a solvent in the fluid reagent is water or a buffer solution.

59. The cancer treatment method according to any one of claims 56 to 58,
wherein the pH of the fluid reagent is 5 to 10.

60. The cancer treatment method according to any one of claims 47 to 59,
wherein the radical generation source includes at least one selected from the group consisting of halogen ions, hypohalous acid ions, halous acid ions, halogen acid ions, and perhalogen acid ions.

61. The cancer treatment method according to claim 60,
wherein the halous acid ions are chlorite ions.

62. The cancer treatment method according to any one of claims 47 to 61,
wherein the cancer cells are epithelial carcinoma cells, and the cancer is epithelial carcinoma.

63. The cancer treatment method according to claim 62,
wherein the epithelial carcinoma is cancer of at least one tissue selected from the group consisting of the urinary system, the reproductive system, the digestive system, the circulatory system, and the respiratory system.

64. The cancer treatment method according to claim 62 or 63,
wherein the epithelial carcinoma is at least one cancer of tissue selected from the group consisting of bladder cancer, uterine cancer, cervical cancer, ovarian cancer, carcinoma of a fallopian tube, testis cancer, prostate cancer, colon cancer, small intestinal cancer, duodenal cancer, gastric cancer, esophageal cancer, laryngeal cancer, oral cancer, tongue cancer, pharyngeal cancer, liver cancer, pancreatic cancer, gallbladder cancer, spleen cancer, peritoneal cancer, lung cancer, and eye cancer.

65. The cancer treatment method according to any one of claims 47 to 64,
wherein the radical generation source is the radical generation source contained in the cancer treatment drug according to any one of claims 29 to 46, and
the cancer treatment method further comprises an administration step of administering the cancer treatment drug to a patient.

66. A method of using the cancer treatment drug according to any one of claims 29 to 46 in cancer treatment.

67. The method according to claim 66, comprising:
an administration step of administering the cancer treatment drug to a patient.

68. A method of using the radical generation source to manufacture the cancer treatment drug according to any one of claims 29 to 46.
